# EUROPEAN PATENT SPECIFICATION

(11) **EP 1 558 255 B1**
(45) Date of publication and mention of the grant of the patent: **27.12.2006**
(21) Application number: 03751019.5
(22) Date of filing: 07.10.2003
(51) Int. Cl.: A61K 31/473, C07D 219/12, C07D 401/12, C07D 417/12

(54) **DUAL BINDING SITE ACETYLCHOLINESTERASE INHIBITORS FOR THE TREATMENT OF ALZHEIMER'S DISEASE**
DUAL BINDENDE ACETYLCHOLINESTERASE INHIBITOREN ZUR BEHANDLUNG VON ALZHEIMER
INHIBITEURS D'ACETYLCHOLINESTERASE A SITE DE LIAISON DOUBLE DESTINES AU TRAITEMENT DE LA MALADIE D'ALZHEIMER

(30) Priority: 09.10.2002 GB 0223494
(43) Date of publication of application: 03.08.2005
(73) Proprietor: Neuropharma, S.A., 28003 Madrid (ES)
(72) Inventor: MARTINEZ GIL, Ana, E-28004 Madrid (ES); DORRONSORO DIAZ, Isabel, E-28005 Madrid (ES); RUBIO ARRIETA, Laura, E-28024 Madrid (ES); ALONSO GORDILLO, Diana, E-28230 Las Rozas - Madrid (ES); FUERTES HUERTA, Ana, E-28003 Madrid (ES); MORALES-ALCELAY, Susana, E-28660 Madrid (ES); DEL MONTE MILLAN, Maria, E-28035 Madrid (ES); GARCIA PALOMERO, Esther, E-28911 Madrid (ES); USAN EGEA, Paola, E-28430 Madrid (ES); DE AUSTRIA, Celia, E-28030 Madrid (ES); MEDINA PADILLA, Miguel, E-28029 Madrid (ES)
(74) Representative: Ruffles, Graham Keith
(86) International application number: PCT/GB2003/004314
(87) International publication number: WO 2004/032929

(56) References cited:
- WO-A-01/17529
- HU, MING-KUAN ET AL: "Homodimeric Tacrine Congeners as Acetylcholinesterase Inhibitors" JOURNAL OF MEDICINAL CHEMISTRY, vol. 45, 26 April 2002 (2002-04-26), pages 2277-2282, XP002267304
- CASTRO ET AL: "Peripheral and Dual Binding Site Acetylcholinesterase Inhibitors: Implications in treatment of Alzheimer's Disease" MINI REVIEWS IN MEDICINAL CHEMISTRY, vol. 1, 2001, pages 267-272, XP001157056 cited in the application
- DATABASE CA [Online] CHEMICAL ABSTRACTS SERVICE, COLUMBUS, OHIO, US; YOUSSEF, KHAIRIA M.: "Synthesis and pharmacological screening of certain substituted tetrahydropyrido[4,3-b]quinolines and pyrazolo[4,3-c]quinolines" XP002267190 retrieved from STN Database accession no. 136:118414 & AL-AZHAR BULLETIN OF SCIENCE (1999), 10(1), 99-112,
- DATABASE CA [Online] CHEMICAL ABSTRACTS SERVICE, COLUMBUS, OHIO, US; YOUSSEF, KHAIRIA M. ET AL: "Synthesis and pharmacological screening of certain substituted tetrahydropyrido [4,3-b] quinolines and pyrazolo [4,3-c] quinolines" XP002267193 retrieved from STN Database accession no. 125:1080 & BULLETIN OF THE FACULTY OF PHARMACY (CAIRO UNIVERSITY) (1995), 33(1), 33-9,

## Description

### FIELD OF THE INVENTION

The invention relates to a novel family of compounds which behaves as dual site acetylcholinesterase inhibitors, as well as to the synthesis and biological evaluation of the compounds of said family. These compounds are especially useful for the treatment of cognitive disorders as senile dementia, cerebrovascular dementia, mild cognition impairment, attention deficit disorder, and/or neurodegenerative dementing disease with aberrant protein aggregations as specially Alzheimer's disease, Parkinson disease, ALS, or prion diseases, as Creutzfeldt-Jakob disease or Gerstmann-Straussler-Scheinher disease. Thus, the invention also relates to pharmaceutical compositions containing said compounds.

### BACKGROUND OF THE INVENTION

Alzheimer's disease (AD) is a progressive neurodegenerative disorder which is one of the most common causes of mental deterioration in elderly people, accounting for about 50-60 % of the overall cases of dementia among persons over 65 years of age. Demographic data indicate that the percentage of elderly in the population is increasing.

Brain regions that are associated with higher mental functions, particularly the neocortex and hippocampus, are those most affected by the characteristic pathology of AD. This includes the extracellular deposits of β-amyloid (derived from amyloid precursor protein, APP) in senile plaques], intracellular formation of neurofibrillary tangles (containing an abnormally phosphorylated form of a microtubule associated protein, tau), and the loss of neuronal synapsis and pyramidal neurons.

The past two decades have witnessed a considerable research effort directed towards discovering the cause of AD with the ultimate hope of developing safe and effective pharmacological treatments. Nowadays, research in the knowledge of the pathogenic cascade that characterizes AD has provided a robust framework for new therapeutic intervention targets.

Nevertheless, current treatment approaches in this disease continue being primarily symptomatic, with the major therapeutic strategy based on the cholinergic hypothesis and specifically on acetylcholinesterase (AChE) inhibition. The successful development of these compounds was based on a well-accepted theory that the decline in cognitive and mental functions associated with AD is related to the loss of cortical cholinergic neurotransmission. This link between cholinergic dysfunction in the basal-cortical system and cognitive deficits has focused scientific efforts on developing tools to elucidate the neurobiological role of the cholinergic system in cognition and to elucidate therapeutic interventions in the disorder. As result, over last decade, the cholinergic hypothesis of AD has launched on the market various cholinergic drugs primarily AChE inhibitors as tacrine, donepezil or rivastigmine, and more recently galanthamine, indicated modest improvement in the cognitive function of Alzheimer's patients.

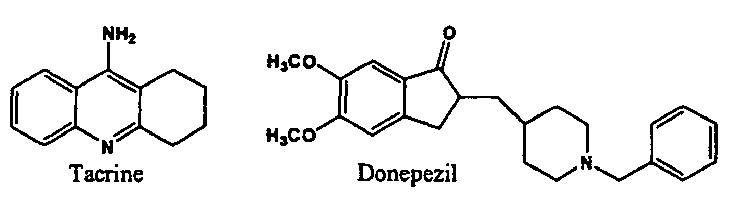

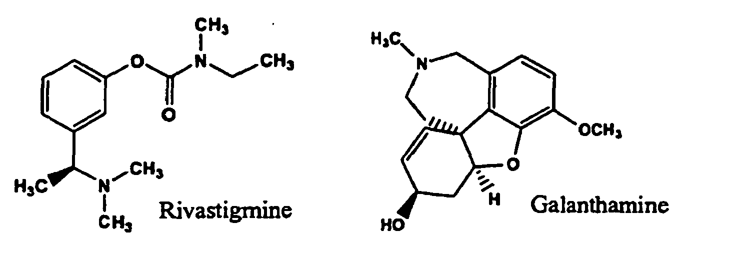

The three dimensional structure of AChE, as determined by x-ray crystallography, revealed that its active site can apparently be reached only through a deep and narrow catalytic gorge. Inhibitors of AChE act on two target sites on the enzyme, the active site and the peripheral site. Inhibitors directed to the active site prevent the binding of a substrate molecule, or its hydrolysis, either by occupying the site with a high affinity molecule (tacrine) or by reacting irreversibly with the catalytic serine (organophosphates and carbamates). The peripheral site consists of a less well-defined area, located at the entrance of the catalytic gorge. Inhibitors that bind to that site include small molecules, such as propidium and peptide toxins as fasciculins. Bis-quaternary inhibitors as decamethonium and others, simultaneously bind to the active and peripheral sites, thus occupying the entire catalytic gorge.

Parallel to the development of antidementia drugs, research efforts have been focused, among others, on the therapeutic potential of AChE inhibitors to slow the disorder progression. This fact was based on a range of evidence, which showed that AChE has secondary non-cholinergic functions.

New evidence shows that AChE may have a direct role in neuronal differentiation. Transient expression of AChE in the brain during embryogenesis suggests that AChE may function in the regulation of neurite outgrowth and in the development of axon tracts. Additionally, the role of AChE in cell adhesion have been studied. The results indicate that AChE promotes neurite outgrowth in neuroblastoma cell line through a cell adhesive role. Moreover, recent studies have shown that the peripheral anionic site of the AChE is involved in the neurotrophic activity of the enzyme and conclude that the adhesion function of AChE is located at the peripheral anionic site. This finding has implications, not only for our understanding of neural development and its disorders, but also for the treatment of neuroblastoma, the leukemias, and especially for Alzheimer's disease.

As it has been previously mentioned, senile plaques are one of pathological hallmarks in AD in which their main component is βA peptide. This is found as an aggregated poorly soluble form. In contrast soluble βA is identified normally circulating in human body fluids. Structural studies of βA showed that synthetic peptides containing the sequences 1-40 and 1-42 of βA can adopt two major conformational states in solution: an amyloidogenic conformer (βA ac) with a high content of β-sheet and partly resistant to proteases and a non-amyloidogenic conformer (βA nac) with a random coil conformation or α-helix and protease-sensitive. AChE colocalizes with βA peptide deposits present in the brain of Alzheimer's patients. It is postulated that AChE binds to a βA nac form acting as a "pathological chaperone" and inducing a conformational transition from βA nac into βA ac in vitro and therefore to amyloid fibrils. AChE directly promotes the assembly of βA peptide into amyloid fibrils forming stable βA-AChE complexes. These complexes are able to change the biochemical and pharmacological properties of the enzyme and cause an increase in the neurotoxicity of the βA fibrils. Moreover, the interaction between these two molecules to form the complex was confirmed by crosslinking experiments. Different studies concerned to the establishment of the binding site of AChE on Aβ have suggested that hydrophobic interactions may play a role in the stabilization of the βA-AChE complex probably due to specific binding to peripheral sites.

Considering the non-cholinergic aspects of the cholinergic enzyme AChE, their relationship to Alzheimer's hallmarks and the role of the peripheral site of AChE in all these functions, an attractive target for the design of new antidementia drugs emerged. Peripheral or dual site inhibitors of AChE may simultaneously alleviate the cognitive deficit in Alzheimer's patients and what it is more important, avoid the assembly of beta-amyloid which represents a new way to delay the neurodegenerative process.

As revealed by the crystallographic structure of AChE and their inhibitors complexes, the AChE active site contains a catalytic triad (Ser 200, His 440, Glu 327) located at the bottom of a deep and narrow gorge, lined with aromatic residues and a subsite, including Trp 84, located near the bottom of the cavity. Trp 84 has been identified as the binding site of the quaternary group of acetylcholine, decamethonium and edrophonium. In addition, Trp 279 at the peripheral site, located at the opening of the gorge, is involved in the binding of the second quaternary group of decamethonium being responsible for the adhesion function of the enzyme.

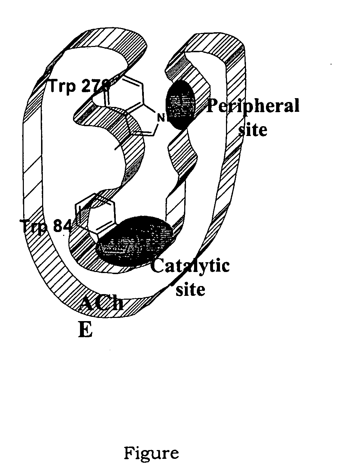

These residues (Trp 84 and 279) have been the basis of the design of a new generation of AChE inhibitors. Thus, ligands able to interact simultaneously with active and peripheral sites could implicate several advantages over the known inhibitors. On one hand, they should improve greatly the inhibitory potency and on the other hand they should be involved in neurotrophic activity. Reference is made to the accompanying Figure. The Article by HU, MING-KUAN et al. in Journal of Medicinal Chemistry Vol. 45, 26 April 2002, 2277-2282 relates to "Homodimeric Tacrine Congeners as Acetylcholinesterase inhibitors".

### SUMMARY OF THE DRAWING

The Figure is a model of the catalytic gorge of AChE

Derivatives that in view of their chemical structure might be classified as dual AchE inhibitors have been reviewed, see Castro, A.; Martinez, A. *Mini Rev. Med. Chem.,* 2001, *1,* 267-272. One of the compounds therein reported, a bis-galanthamine inhibitor, is recently described by molecular modelling techniques as a bis-functional ligand for the AchE, see Luttmann, E.; Linnemann, E.; Fels, G. *J. Mol. Model.,* 2002, *8*, 208-216.

The following compounds might also be classified as dual AchE inhibitors:

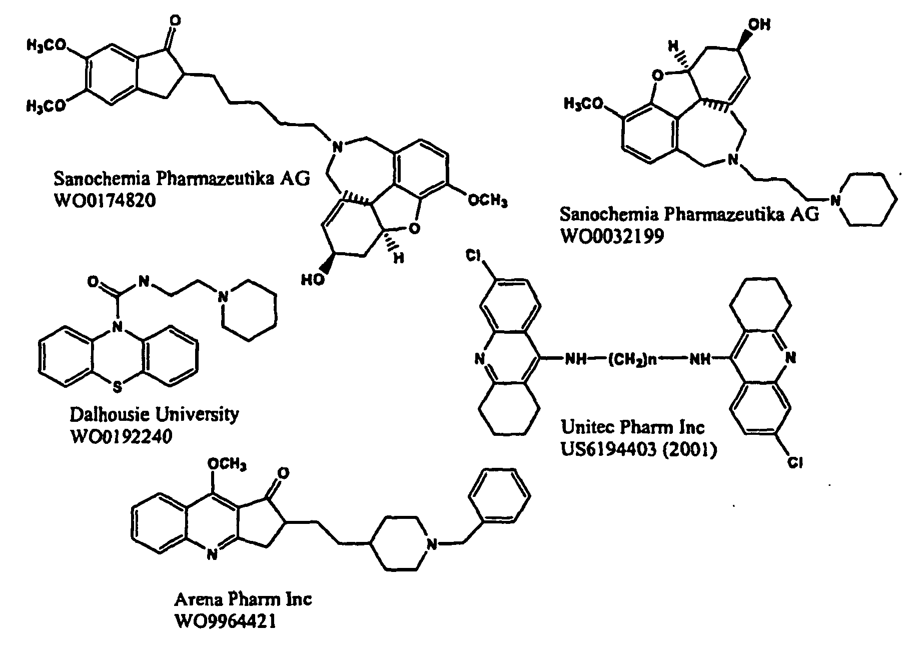

In particular, US Patent 6,194,403 describes bis-halo-tacrinylalkanes for use in the treatment of Alheimer's disease.

### SUMMARY OF THE INVENTION

We have applied this new concept for the design of dual AchE inhibitors, in order to have compounds that show potent AchE inhibition activities together with modifications in the β-amyloid aggregation properties.

In particular, after extensive research, we have developed a new family of compounds which show potent AchE inhibition activities together with modifications in the β-amyloid aggregation properties.

The invention discloses a novel family of compounds which behaves as dual site acetylcholinesterase inhibitors, as well as to the synthesis and biological evaluation of the compounds of said family. These compounds are especially useful for the treatment of cognitive disorders as senile dementia, cerebrovascular dementia, mild cognition impairment, attention deficit disorder, and/or neurodegenerative dementing disease with aberrant protein aggregations as specially Alzheimer's disease, Parkinson disease, ALS, or prion diseases, as Creutzfeldt-Jakob disease or Gerstmann-Straussler-Scheinher disease. The invention also relates to pharmaceutical compositions containing said compounds.

### DETAILED DESCRIPTION OF THE INVENTION

The invention is directed to the compounds represented by the general formula I 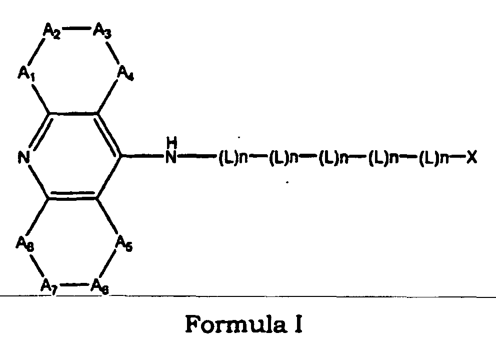 wherein:
- X: is the following radical 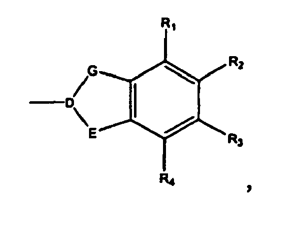
- L: is independently selected from -C(R)(R'')-, -CO-, -O- or -NR'-
- n: is zero, one, two, three, four, five, six, seven, eight, nine or ten
- R and R'': are independently selected from hydrogen, alkyl, aryl,heteroaryl, halo, haloalkyl, alkoxy, hydroxyl, nitro and alkylthio
- D: is independently selected from -C(R₉)-, =C-, or -N-
- A₁, A₂, A₃, A₄, A₅, A₇, A₈,: G and E are independently selected from -CO-, -C(R₁₀)(R₁₁)-, =C(R₁₀)-, -N(R₁₂)-, =N-, -O-, -S(O)ₜ-
- R₁, R₂, R₃, R₄, R₉, R₁₀ and R₁₁: are independently selected from hydrogen, alkyl, alkoxy, hydroxyl, alkylthio, cycloalkyl, haloalkyl, halo, aryl, -(Z)ₙ-, aryl, heteroaryl, -O(R₇), -C(O)R₇, -C(O)OR₇, -S(O)ₜ, cyano, nitro and mercapto, aryl substituted by alkyl, alkoxy, hydroxy, halo, haloalkyl, nitro or alkylthio; and heteroaryl substituted by alkyl, alkoxy, hydroxy, halo, haloalkyl, nitro or alkylthio
- R₁₂: is independently selected from hydrogen, alkyl, alkoxy, hydroxyl, cycloalkyl, haloalkyl, aryl, heteroaryl, aryl substituted by alkyl, alkoxy, hydroxy, halo, haloalkyl, or alkylthio; and heteroaryl substituted by alkyl, alkoxy, hydroxy, halo, haloalkyl, nitro or alkylthio
- Z: is independently selected from -C(R₇)(R₈)-, -C(O)-, -O-, -C(=NR₇)-, -S(O)ₜ, N(R₇)-
- R₇ and R₈: are independently selected from hydrogen, alkyl, alkoxy, alkylthio, cycloalkyl, haloalkyl, halo, aryl, heteroaryl, cyano, nitro, mercapto, aryl substituted by alkyl, alkoxy, hydroxy, halo, haloalkyl, nitro or alkylthio; and heteroaryl substituted by alkyl, alkoxy, hydroxy, halo, haloalkyl, nitro or alkylthio
- t: is zero, one or two,
with the exclusion of the following two compounds:

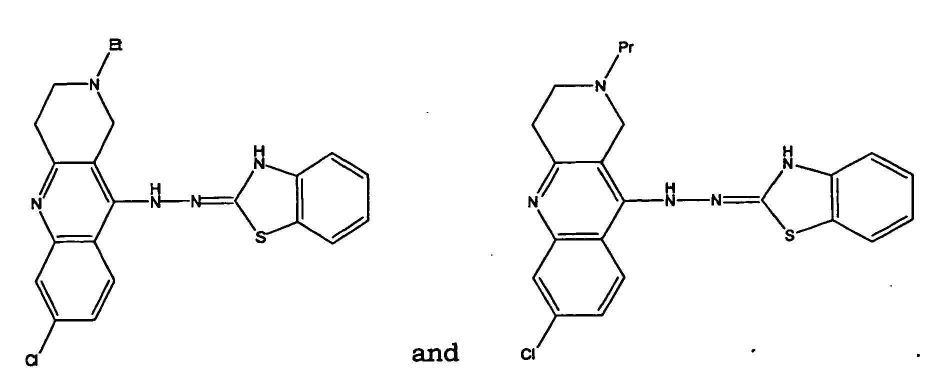

Preferably, the compounds within the formula (I) are represented by the formula Ia

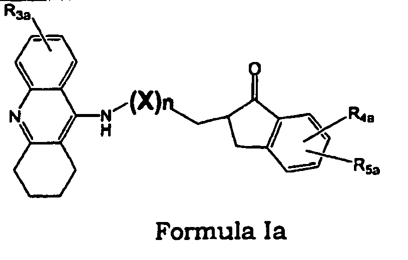

Where:
- X: is -C(R^{1a})(R^{2a})-, -CO-, -O- or -NR^{1a-};
- n: is zero, one, two, three, four, five, six, seven, eight, nine or ten;
- R^{1a} and R^{2a}: are independently selected from hydrogen, alkyl, aryl, halo, haloalkyl;
- R^{3a}, R^{4a} and R^{5a}: are independently selected from hydrogen, alkyl, cycloaklyl, haloalkyl, halo, aryl, -(Z)ₙ-aryl, heteroaryl, -OR^{3a}, -C(O)R^{3a}, -C(O)OR^{3a}, -S(O)ₜ-;
- t: is zero, one or two;
- Z: is independently selected from C(R^{3a})(R^{4a})-, -C(O)-, -O-, -C(=NR^{3a})-, -S(O)ₜ-, N(R^{3a})-.

### Definitons

Unless otherwise specified, the following terms have the following meaning:;
- "alkyl": refers to a straight-line or branched hydrocarbon chain comprising only atoms of carbon and hydrogen and containing no unsaturated bonds, having from one to eight carbon atoms and bound to the remainder of the molecule by a single bond, e.g. methyl, ethyl, n-propyl, i-propyl, n-butyl, t-butyl, n-pentyl, etc. The alkyl radicals may optionally be substituted by one or more substituents chosen independently from the group comprising halogens, hydroxyl, alcoxides, carboxy, cyano, carbonyl, acyl, alkoxycarbonyl, amino, nitro, mercapto and alkylthio. Preferably, alkyl is C₁-C₆ alkyl.
- "alkoxy": refers to a radical of formula -ORₐ, where Rₐ is an alkyl radical as described above, e.g. methoxy, ethoxy, propoxy, etc.
- "alkoxycarbonyl": refers to a radical of formula -C(O)ORₐ, where Rₐ is an alkyl radical as described above, e.g. methoxycarbonyl, ethoxycarbonyl, propoxycarbonyl, etc.
- alkylthio": refers to a radical of formula -SRa, where Ra is an alkyl radical as described above, e.g. methylthio, ethylthio, propylthio, etc.
- "amino": refers to a radical of formula -NH2
- "aryl": refers to a phenyl or naphthyl radical. The aryl radical may optionally be substituted by one or more substituents selected from among the group comprising hydroxy, mercapto, halogens, alkyl, phenyl, alkoxy, haloalkyl, nitro, cyano, dialkylamino, aminoalkyl, acyl and alkoxycarbonyl as they are defined here.
- "acyl": refers to a radical of formula -C(O)-Rₐ and -C(O)-R_{b}, where Rₐ is an alkyl radical as described above and R_{b} is an aryl radical as described above, e.g. acetyl, propionyl, benzoyl, and similar.
- "carboxy": refers to a radical of formula -C(O)OH
- "cyano": refers to a radical of formula -CN
- "cycloalkyl": refers to stable cycles of 3 to 10 monocyclic or bicyclic members that are saturated or partially saturated and consist exclusively of carbon and hydrogen atoms. This term also includes cycloalkylo radicals, which may optionally be substituted by one or more substituents chosen independently from the group comprising alkyl, halogen, hydroxy, amino, cyano, nitro, alkoxy, carboxy and alkoxycarbonyl
- "halogens": refers to bromine, chlorine, iodine or fluorine
- "haloalkyl": refers to an alkyl radical, as defined above, which is substituted by one or more halogens, also as defined above, e.g. trifluoromethyl, trichloromethyl, 2,2,2-trifluoroethyl, 1-fluoromethyl-2-fluoroethyl, and similar.
- "heterocycle": refers to a heterocyclic radical. The heterocycle refers to a stable cycle of 3 to 15 members comprising carbon atoms and one to five heteroatoms chosen from the group comprising nitrogen, oxygen and sulphur. For the purposes of this invention, the heterocycle may be a monocyclic, bicyclic or tricyclic system that may include fused rings, and the nitrogen, carbon or sulphur atoms may optionally be oxidised, the nitrogen atom may optionally be quaternised, and the heterocycle may be partly or totally saturated or aromatic. Examples of these heterocycles include, but are not limited to, azepines, benzimidazole, benzothiazole, furan, isothiazole, imidazole, indole, piperidine, piperazine, purine, quinoline, thiadiazole, tetrahydrofuran. The heterocycle may optionally be substituted by R₃ and R₄ as defined in the summary of the invention.
- "mercapto": refers to a radical of formula -SH
- "nitro": refers to a radical of formula -NO₂.

In the chain -(L)n-(L)n-(L)n-(L)n-(L)n-, the or each group -(L)ₙ- is preferably -(CH₂)ₙ- (where n is not zero), -CO- -NH- or -NCH₃-. Preferably there are at least one or two groups -(L)n- where n is not zero. Suitably the chain is of the formula -(CH₂)ₙ-, -(CH₂)ₙ-NRₐ-(CH₂)ₙ-, -(CH₂)ₙ-NRₐ-CO-, -(CH₂)ₙ-NRₐ-CO-(CH₂)ₙ- or -(CH₂)ₙ-NRₐ-(CH₂)ₙ-NRₐ-CO-, where the or each n is not zero, and the or each Rₐ is -NH- or -NCH₃-, usually preferably -NH-. The total for the sum of the n integers is preferably in the range 2 to 15.

In the formula: 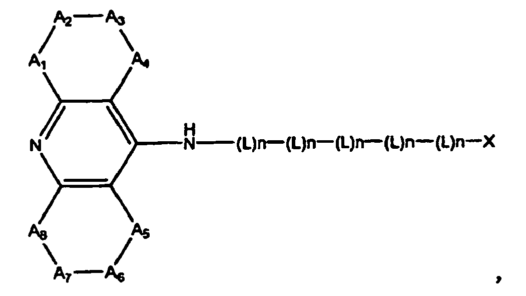 each A group (that it, A₁ to A₈) is preferably =CH- or -CH₂-, though one or both of A₂ and A₇ can be halo, especially chloro, when the remaining A groups are =CH-.

In formula I, preferably D is -CH-, =C- or -N-. Preferably E is -CO-, -CH₂-. =CH-, =N- -O- or -S-. Preferably G is -CO-, -CH₂-. =CH-, or =N-. Preferably R₁ to R₄ are hydrogen.

Especially preferred within these compounds are those in which X is: phthalimidyl (1,3-dioxo-1,3-dihydro-isoindol-2-yl), indol-2-yl, indanon-2-yl, benzimidazol-2-yl, indandion-2-yl, indazol-2-yl, benzofuran-2-yl, benzothiophen-2-yl or benzotriazol-2-yl.

More preferred compounds are those in which X is phthalimide (1,3-dioxo-1,3-dihydro-isoindol-2-yl) and the cyclic part of formula I represents 9-acridinyl, 1,2,3,4-tetrahydro-acridin-9-yl or 6-chloro,1,2,3,4-tetrahydro-acridin-9-yl. Some preferred compounds are:
- 2-[6-(acridin-9-ylamino)-hexyl]-isoindole-1,3-dione (6),
- 2-[7-(acridin-9-ylamino)-heptyl]-isoindole-1,3-dione (7),
- 2-[8-(acridin-9-ylamino)-octyl]-isoindole-1,3-dione (8),
- 2-[9-(acridin-9-ylamino)-nonyl]-isoindole-1,3-dione (9),
- N-[7-(6-Chloro-1,2,3,4-tetrahydro-acridin-9-ylamino)-heptyl]-2-(1,3-dioxo-1,3-dihydro-isoindol-2-yl)-acetamide (10),
- N-(3-{[3-(6-Chloro-1,2,3,4-tetrahydro-acridin-9-ylamino)-propyl]-methyl-amino}-propyl)-2-(1,3-dioxo-1,3-dihydro-isoindol-2-yl)-acetamide (11),
- N-[6-(6-Chloro-1,2,3,4-tetrahydro-acridin-9-ylamino)-hexyl]-2-(1,3-dioxo-1,3-dihydro-isoindol-2-yl)-acetamide (12),
- 2-[6-(1,2,3,4-Tetrahydro-acridin-9-ylamino)-hexylamino]-indan-1,3-dione (3),
- 2-[7-(1,2,3,4-Tetrahydro-acridin-9-ylamino)-heptyl]-isoindole-1,3-dione (4), and
- 2-[8-(1,2,3,4-Tetrahydro-acridin-9-ylamino)-octyl]-isoindole-1,3-dione (5)

More preferred compounds are those in which X is 1-indanon-2-yl and the cyclic part of formula I represents 1,2,3,4-tetrahydro-acridin-9-yl; within these compounds are included, among others, the following compounds:
- 5,6-Dimethoxy-2-{[7-(1,2,3,4-tetrahydro-acridin-9-ylamino)-heptylamino]-methyl}-indan-1-one (1), and
- 5,6-Dimethoxy-2-{[6-(1,2,3,4-tetrahydro-acridin-9-ylamino)-hexylamino]-methyl}-indan-1-one (2)

Another object of the invention is the synthesis of the compounds of the invention.

The synthesis of the compounds follow a convergent pathway strategy that could be summarized in schemes 1a and 1b,

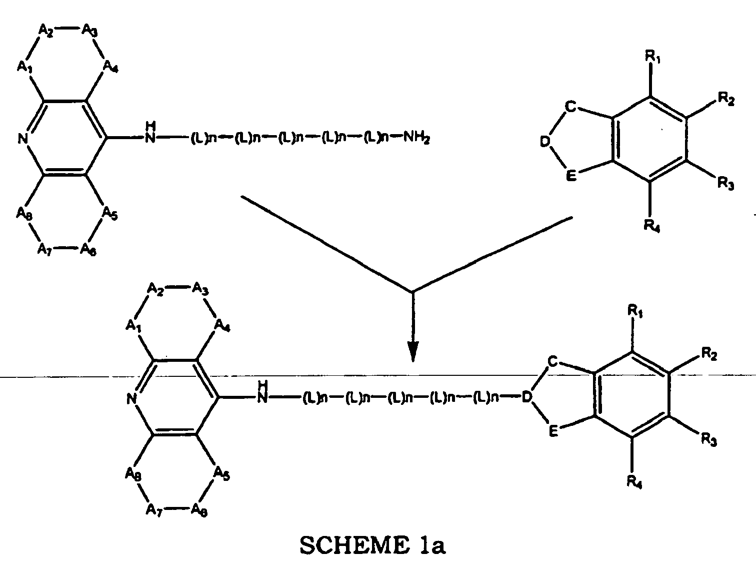

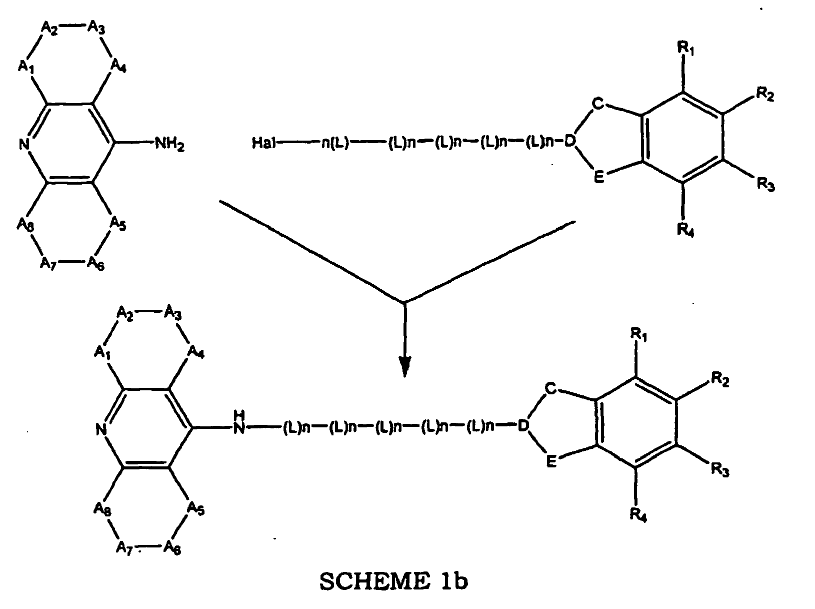

9-Alkylaminotetrahydroacridines have been synthesized following the procedure previously reported in bibliography. Carlier, P.R.; Chow. E.S.-H; Han, Y.; Liu, J.; El Yazal, J.; Pang Y.-P. *J. Med. Chem.,* 1999,*42*, 4225-4231.

Specific examples are:

### Example 1: 5,6-Dimethoxy-2-{[7-(1,2,3,4-tetrahydro-acridin-9-ylamino)-heptylamino]-methyl}-indan-1-one.

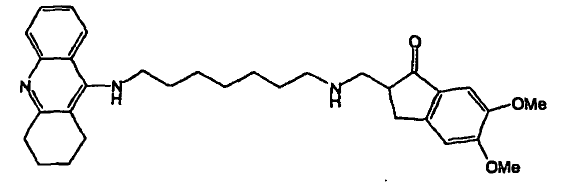

To a stirred solution of 9-(7-aminoheptylamino)- 1,2,3,4-tetrahydroacridine (134 mg, 0.43 mmol) in a mixture of ethanol: water 3:1 (3.5 ml) at room temperature, paraformaldehyde (26 mg, 0.86 mmol) and 5,6-dimethoxyindan-1-one (83 mg, 0.43 mmol) were added. The pH was adjusted to 3 with 35% hydrochloric acid and the mixture were refluxed for 24 hours. At the end of this period, the reaction mixture was cooled (25°C), the solvent was removal under vacuum pressure and the residue was treated with K₂CO₃ saturated solution (3.5 ml) and methylene chloride (5 ml). The organic layer was washed with water (5 ml) and dried (anhydrous Na₂SO₄). The solvent was removed under vacuum and the residue was purified by preparative centrifugal thin layer chromatography. Elution with 5: 1 ethyl acetate: methanol containing 1% of aquous ammonia afforded the title compound as yellow syrup (15 mg, 6.8 %).
¹H-NMR (CDCl₃, 300MHz, δ): 7.93 (dd, 2H, *J*=8.2 Hz), 7.53 (ddd,, 1H, *J*=8.2, 1.3 Hz), 7.32 (ddd, , 1H, *J*=8.2, 1.3 Hz), 7.13 (s, 1H), 6.85 (s, 1H), 3.94 (s, 3H), 3.88 (s, 3H), 3.48 (t, 2H, *J*=7.1 Hz), 3.28-3.19 (m, 1H), 3.10-3.05 (m, 2H), 2.89-2.56 (m, 9H), 1.91-1.88 (m, 4H), 1.63 (quint, 2H, *J*=7.5 Hz), 1.50-1.37 (m, 2H), 1.34-1.32 (m, 6H).
¹³C-NMR (CDCl₃, 300MHz, δ): 203.0, 155.9, 151.3, 149.7, 149.6, 129.6, 128.8, 128.4, 123.9, 123.2, 107.6, 104.4, 56.5, 56.4, 51.5, 50.1, 49.7, 33.9, 31.9, 31.6, 30.0, 29.5, 27.4, 27.1, 27.0, 24.9, 23.2, 22.9.
ESI-MS: m/z [M+H⁺]⁺ 516.

### Example 2: 5,6-Dimethoxy-2-{[6-(1,2,3,4-tetrahydro-acridin-9-ylamino)-hexylamino]-methyl}-indan-1-one.

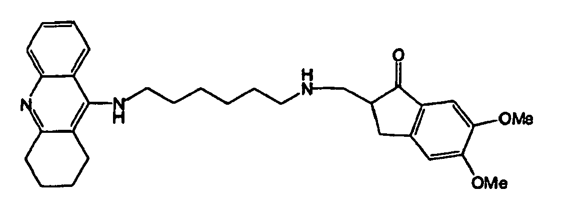

According to the general procedure in Example 1, 9-(6-aminohexylamino)-1,2,3,4-tetrahydroacridine (96 mg, 0.32 mmol), paraformaldehyde (19 mg, 0.64 mmol), 5,6-dimethoxyindan-1-one (62 mg, 0.32 mmol) and 35% hydrochloric acid (pH=3) were refluxed for 24 hours. Purification by two preparative centrifugal thin layer chromatographies eluting with 10: 1 ethyl acetate: methanol containing 2% of aquous ammonia afforded the title compound as yellow syrup (8 mg, 5 %).
¹H-NMR (CDCl₃, 300MHz, δ): 7.97 (dd, 2H, *J*=8.1 Hz), 7.56 (ddd, , 1H, *J*=8.1, 1.2 Hz), 7.34 (ddd, , 1H, *J*=8.2, 1.2 Hz), 7.13 (s, 1H), 6.85 (s, 1H), 3.94 (s, 3H), 3.88 (s, 3H), 3.55 (t, 2H, *J*=7.0 Hz), 3.30-3.19 (m, 1H), 3.10-3.07 (m, 2H), 2.90-2.60 (m, 7H), 1.89-1.68 (m, 4H), 1.40-1.35 (m, 2H), 1.28-1.11 (m, 6H).
¹³C-NMR (CDCl₃, 300MHz, δ): 207.1, 155.7, 151.2, 149.4, 149.3, 129.3, 128.7, 128.3, 123.8, 123.0, 107.4, 104.2, 56.3, 56.1, 51.3, 49.7, 49.4, 47.3, 31.6, 31.3, 29.7, 27.0, 24.6, 22.9, 22.5.
ESI-MS: m/z [M+H⁺]⁺ 502.

### Example 3. 2-[6-(1,2,3,4-Tetrahydro-acridin-9-ylamino)-hexylamino]-indan-1,3-dione

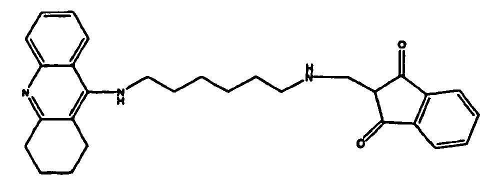

To a stirred solution of (1,2,3,4-Tetrahydro-acridin-9-yl)-hexane-1,6-diamine oxalate (339 mg, 0.87 mmol) in a mixture of ethanol: water 3:1 (3.5 ml) at room temperature, paraformaldehyde (26,4 mg, 0.88 mmol) and Indan-1,3-dione (129 mg, 0.88 mmol) were added. The pH was adjusted to 3 with 35% hydrochloric acid and the mixture were refluxed for 24 hours. At the end of this period, the reaction mixture was cooled (25°C), the solvent was removal under vacuum pressure and the residue was treated with K₂CO₃ saturated solution (3.5 ml) and methylene chloride (5 ml). The organic layer was washed with water (5 ml) and dried (anhydrous Na₂SO₄). The solvent was removed under vacuum and the residue was purified by preparative centrifugal thin layer chromatography. Elution from 10:1 to 3:1 ethyl acetate: methanol containing 1% of aquous ammonia afforded the title compound as yellow syrup (17 mg, 4.4 %).
¹H-NMR (CDCl₃, 400MHz, δ):7.92 (m, 4H), 7.84 (dd , 2H, *J*=6, *J*=2.8, Hz), 7.54 (m, 1H), 7.32 (m, 1H), 3.70(t, 2H, *J*=6.4), 3.60 (m1H), 3.50 (t, 2H, *J*=6.4), 3.08 (m, 2H), 2.65 (m, 2H), 31.8-2.0(m, 4H), (1.80, 2H, m), (1.70, m, 2H), (1.45, m,4H)
¹³C-NMR (CDCl₃, 300MHz, δ): 200.0, 159.0, 151.3, 147.1, 140.6, 136.8, 136.1, 130.5, 129.9, 123.6, 123.0, 120.8, 120.4, 53.1, 52.9, 49.3, 48.9, 31.4, 29.8, 26.7, 26.4, 24.7, 22.6, 22.0.
ESI-MS: m/z [M+H⁺]⁺442.

### General method for the synthesis of isoindol derivatives (Scheme 4, Examples 4-9)

To a solution of KOH in DMSO was added 9-Amino-1,2,3,4-tetrahydroacridine under N₂, and the mixture was stirred for 4 hours at room temperature. After this time the brominated alkyl isoindol derivative was added and the resulting orange solution was stirred for 12 hours at room tempereture, and then the solvent was eliminated washing with water and extracted with ethyl acetate. The combined organic extracts were washed with NaCl solution, and then were dried with Na₂SO₄ anhydrous. The solvent was evaporated under reduced pressure and the residue purified by silica gel column chromatography using as eluent mixtures of solvents in the proportions indicated for each case.

The brominated alkyl isoindol derivatives have been synthesized following the procedure previously reported in bibliography: Donahoe et al, J. Org.Chem, 22, 1957, 68.

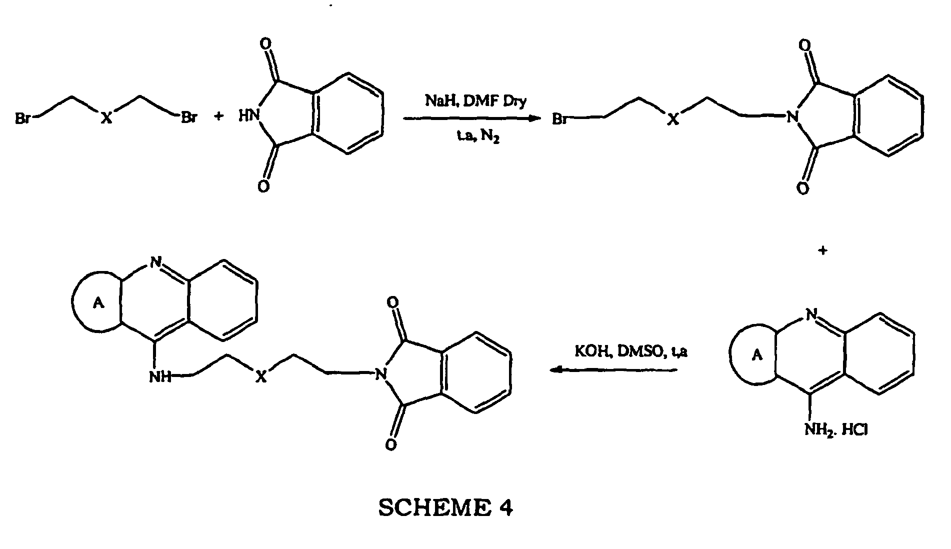

### Example 4. 2-[7-(1,2,3,4-Tetrahydro-acridin-9-ylamino)-heptyl]-isoindole-1,3-dione

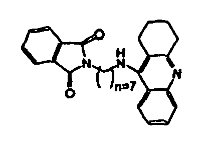

Reagents: 9-Amino-1,2,3,4-tetrahydroacridine (100 mg, 0.42 mmol), DMSO (5 ml), KOH (47 mg, 0.8 mmol), and 2-(7-Bromo-heptyl)-isoindole-1,3-dione (278 mg, 0.8 mmol).
Purification: silica gel column chromatography using DCM/ MeOH/ NH₃ (10:1:0.5%). Yellow syrup, yield: 17 mg (5%).
¹H-NMR (CDCl₃, 400MHz, δ ppm): 8.41 (brs, 1H), 8.12 (d, 1H, *J*=8.6 Hz), 7.82 (dd, 2H, *J*=5.0 Hz, *J*=2.7 Hz), 7.68 (dd, 3H, *J*=5.0 Hz, *J*=2.7 Hz), 7.43 (t, 1H, *J*=8.6 Hz), 3.83 (brs, 2H), 3.65 (t, 2H, *J*=7 Hz), 3.25 (brs, 2H), 2.61 (t, 2H, *J*=5.8 Hz), 1.97-1.92 (m, 4H), 1.84-1.80 (m ,2H), 1.78-1.72 (m, 2H), 1.47-1.43 (m , 6H).
¹³C-NMR (CDCl₃, 100MHz, δ ppm): 168.5, 166.5, 154.0, 133.8, 132.5, 132.0, 131.2, 128.9, 125.3, 124.1, 123.3, 119.3, 54.6, 49.1, 38.5, 29.7, 29.2, 28.4, 28.2, 26.5, 25.2, 23.4, 22.9, 22.5, 22.1, 21.1, 14.4.
ESI-MS[M+H⁺]⁺443.

### Example 5. 2-[8-(1,2,3,4-Tetrahydro-acridin-9-ylamino)-octyl]-isoindole-1,3-dione

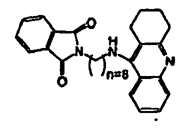

Reagents: 9-Amino-1,2,3,4-tetrahydroacridine (100 mg, 0.42 mmol), DMSO (5 ml), KOH (47 mg, 0.8 mmol), and 2-(8-Bromo-octyl)-isoindole-1,3-dione (240 mg, 0.8 mmol).

Purification: silica gel column chromatography using DCM/ MeOH/ NH₃ (10:1: 0.5%). Yellow syrup, yield: 30 mg (15%).
¹H-NMR (CDCl₃, 400MHz, δ ppm): 8.43 (brs, 1H), 8.12 (d, 1H, *J*=8.6 Hz), 7.82 (dd, 2H, *J*=5.0 Hz, *J*=2.7 Hz), 7.68 (dd, 3H, *J*=5.0 Hz, *J*=2.7 Hz), 7.43 (t, 1H, *J*=8.6 Hz), 3.83 (brs, 2H), 3.65 (t, 2H, *J*=7 Hz), 3.25 (brs, 2H), 2.61 (t, 2H, *J*=5.8 Hz), 1.97-1.92 (m, 4H), 1.84-1.80 (m , 2H), 1.78-1.72 (m, 2H), 1.47-1.43 (m , 8H).
¹³C-NMR (CDCl₃, 100MHz, δ ppm):168.6, 166.2, 154.2, 134.1, 132.6, 132.3, 131.0, 129.0, 125.4, 124.0, 123.3, 119.3, 54.5, 49.0, 38.0, 29.8, 29.2, 28.9, 28.6, 26.7, 25.0, 23.6, 23.0, 22.8, 22.1, 21.0, 14.2.
ESI-MS[M+H⁺]⁺455.

### Example 6. 2-[6-(Acridin-9-ylamino)-hexyl]-isoindole-1,3-dione

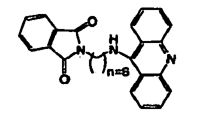

Reagents: 9-Amino-acridina (100 mg, 0.42 mmol), DMSO (5 ml), KOH (47 mg, 0.8 mmol), and 2-(6-Bromo-hexyl)-isoindole-1,3-dione (240 mg, 0.8 mmol).

Purification: silica gel column chromatography using DCM/ MeOH/ NH₃ (10:1:0.5%). Yellow syrup, yield: 30 mg (15%).
¹H-NMR (CDCl₃, 400MHz, δ ppm): 8.08 (d, 2H, *J*=8.6 Hz), 8.02(d, 2H, *J*=8.6 Hz), 7.81 (dd, 2H, *J*=5.4 Hz, *J*=3.1 Hz), 7.68 (dd, 3H, *J*=5.4 Hz, *J*=3.1 Hz), 7.59 (t, 2H, *J*=6.6 Hz), 7.30 (t, 2H, *J*=6.6 Hz) 3.85 (t, 2H, *J*=7 Hz), 3.67 (t, 2H, *J*=7 Hz), 1.83 (q, 2H, *J*=7 Hz), 1.67 (q, 2H, *J*=7 Hz), 1.45-1.34 (m, 6H).
¹³C-NMR (CDCl₃, 100MHz, δ ppm): 168.2, 156.3, 133.8, 133.6, 131.9, 128.7, 128.5, 124.7, 123.0, 122.8, 119.3, 111.9, 48.4, 37.68, 29.81, 26.37, 22.81.
ESI-MS[M+H⁺]⁺424.

### Example 7. 2-[7-(Acridin-9-ylamino)-heptyl]-isoindole-1,3-dione

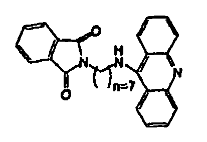

Reagents: 9-Amino-acridine (60 mg, 0.24 mmol), DMSO (5 ml), KOH (27 mg, 0.48 mmol), and 2-(7-Bromo-heptyl)-isoindole-1,3-dione (80mg, 0.24 mmol).

Purification: silica gel column chromatography using DCM/ MeOH (10:1:0.1%NH3). Yellow syrup, yield: 80 mg (74%).
¹H-NMR (CDCl₃, 400MHz, δ ppm): 8.08 (d, 2H, *J*=8.6 Hz), 8.02 (d, 2H, *J*=8.6 Hz), 7.81 (dd, 2H, *J*=5.4 Hz, *J*=3.1 Hz), 7.68 (dd, 3H, *J*=5.4 Hz, *J*=3.1 Hz), 7.59 (t, 2H, *J*=6.6 Hz), 7.30 (t, 2H, *J*=6.6 Hz), 3.85 (t, 2H, *J*=7 Hz), 3.67 (t, 2H, *J*=7 Hz), 1.83 (q, 2H, *J*=7 Hz), 1.67 (q, 2H, *J*=7 Hz), 1.45-1.34 (m, 6H).
¹³C-NMR (CDCl₃, 100MHz, δ ppm): 168.3, 155.3, 133.8, 133.7, 132.7, 132.0, 124.5, 123.0, 122.9, 121.7, 113.2, 49.0, 37.7, 30.4, 28.6, 28.3, 26.6, 26.5.
ESI-MS[M+H⁺]⁺438.

### Example 8. 2-[8-(Acridin-9-ylamino)-octyl]-isoindole-1,3-dione

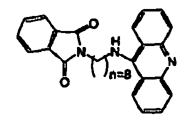

Reagents: 9-Amino-acridina (60 mg, 0.24 mmol), DMSO (5 ml), KOH (27 mg, 0.48 mmol), and 2-(7-Bromo-heptyl)-isoindole-1,3-dione (68.64 mg, 0.24 mmol). Purification: silica gel column chromatography using DCM/ MeOH (10:1:0.1%NH₃). Yellow syrup, yield: 20 mg (18.5%).
¹H-NMR (CDCl₃, 400MHz, δ ppm): 8.06 (d, 2H, *J*=8.6 Hz), 8.02 (d, 2H, *J*=8.6 Hz), 7.80 (dd, 2H, *J*=5.4 Hz, *J*=3.1 Hz), 7.68 (dd, 3H, *J*=5.4 Hz, *J*=3.1 Hz), 7.59 (t, 2H, *J*=6.6 Hz), 7.30 (t, 2H, *J*=6.6 Hz) 3.82 (t, 2H, *J*=7 Hz), 3.65 (t, 2H, *J*=7 Hz), 1.83 (q, 2H, *J*=7 Hz), 1.68 (q, 2H, *J*=7 Hz), 1.45-1.34 (m, 8H)
¹³C-NMR (CDCl₃, 100MHz, δ ppm): 168.3, 155.4, 134.1, 133.7, 132.7, 131.9, 124.6, 123.1, 122.8, 121.7, 113.1, 48.7, 37.6, 30.0, 28.5, 28.1, 26.4, 26.0, 23.2.
ESI-MS[M+H⁺]⁺451.

### Example 9. 2-[9-(Acridin-9-ylamino)-nonyl]-isoindole-1,3-dione

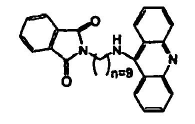

Reagents: 9-Amino-acridine (150 mg, 0.60 mmol), DMSO (10 ml), KOH (67.3 mg, 1.2 mmol), and 2-(9-Bromo-nonyl)-isoindole-1,3-dione (68.64 mg, 0.24 mmol). Purification: silica gel column chromatography using DCM/ MeOH (10:1:0.1%NH3). Yellow syrup, yield: 20 mg (18.5%).
¹H-NMR (CDCl₃, 400MHz, δ ppm): 8.06 (d, 2H, *J*=8.6 Hz), 8.02(d, 2H, *J*=8.6 Hz), 7.80 (dd, 2H, *J*=5.4 Hz, *J*=3.1 Hz), 7.68 (dd, 3H, *J*=5.4 Hz, *J*=3.1 Hz), 7.62 (t, 2H, *J*=6.6 Hz), 7.33 (t, 2H, *J*=6.6 Hz) 3.82 (t, 2H, *J*=7 Hz), 3.65 (t, 2H, *J*=7 Hz), 1.83 (q, 2H, *J*=7 Hz), 1.68 (q, 2H, *J*=7 Hz), 1.45-1.34 (m, 10H).
¹³C-NMR (CDCl₃, 100MHz, δ ppm): 168.3, 155.4, 134.2, 133.6, 132.7, 132.0, 124.2, 123.5, 122.5, 121.3, 113.0, 48.6, 37.6, 30.0, 27.5, 28.1, 26.2, 26.0, 23.2, 22.3.
ESI-MS[M+H⁺]⁺451.

### General method for the synthesis of N-phthaloglycine derivatives (Scheme 5, Examples 10-12)

To a solution of *N*-phthaloglycine in THF anhydrous was added 1,1'-carbonyldiimidazol under N₂, and the mixture was stirred for 4 hours at room temperature. After this time the amine was added and the resulting amber solution was stirred for 20 hours, and then the solvent was evaporated under reduced pressure, water was added and the resulted mixture were extracted with dichlorometane. The combined organic extracts were washed with NaCl solution, and then were dried with Na₂SO₄ anhydrous. The solvent was evaporated under reduced pressure and the residue purified by silica gel column chromatography using as eluent mixtures of solvents in the proportions indicated for each case.

The derivatives of the present invention may be prepared as described below in scheme 5.

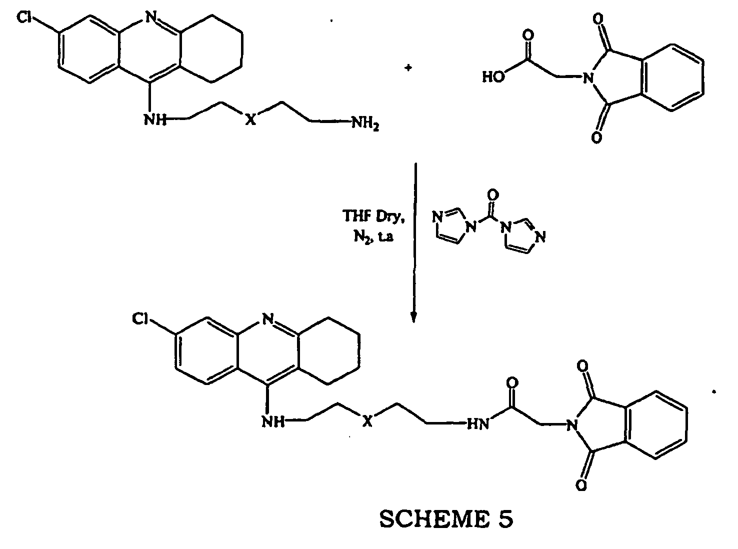

### Example 10.- N-[7-(6-Chloro-1,2,3,4-tetrahydro-acridin-9-ylamino)-heptyl]-2-(1,3-dioxo-1,3-dihydro-isoindol-2-yl)-acetamide

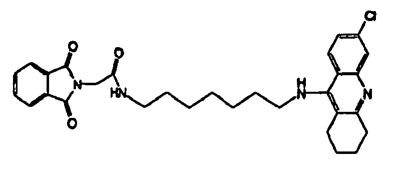

Reagents: *N*-phthaloglycine (83 mg, 0.48 mmol), THF anhydrous (5 ml), 1,1'-carbonyldiimidazol (83 mg, 0.51 mmol), and 6-chloro-9-(7-aminoheptylamino)- 1,2,3,4-tetrahydroacridine (165 mg, 0.48 mmol). Purification: silica gel column chromatography using DCM/ MeOH/ NH₃ (20:1:0.5%). Yellow syrup, yield: 80 mg (31%).
¹H-NMR (CDCl₃, 400MHz, δ ppm): 7.89 (d, 1H, *J*=8.9 Hz), 7.86 (d, 1H, *J*=2.3 Hz), 7.81 (dd, 2H, *J*=5.4 Hz, *J*=3.1 Hz), 7.68 (dd, 2H, *J*=5.4 Hz, *J*=3.1 Hz), 7.23 (dd, 1H, *J*=8.9 Hz, *J*=2.0 Hz), 6.22 (brs, 1H, ), 4.32 (s, 2H), 4.18 (brs, 1H), 3.46-3.48 (m, 2H), 3.24 (c, 2H, *J*=6.6 Hz), 3.02 (brs, 2H), 2.65 (brs, 2H), 1.90 (m, 4H), 1.67-1.63 (m , 2H), 1.50-1.46 (m, 2H), 1.38-1.30(m , 4H).
¹³C-NMR (CDCl₃, 100MHz, δ ppm): 167.7, 166.3, 157.2, 152.0, 145.4, 135.1, 134.0, 131.6, 124.8, 124.6, 124.4, 123.2, 117.0, 114.3, 49.1, 40.5, 39.6, 32.1, 31.2, 29.1, 28.6, 26.5, 26.4, 24.3, 22.5, 22.0.
ESI-MS[M+H⁺]⁺533.

### Example 11.- N-(3-{[3-(6-Chloro-1,2,3,4-tetrahydro-acridin-9-ylamino)-propyl]-methyl-amino}-propyl)-2-(1,3-dioxo-1,3-dihydro-isoindol-2-yl)-acetamide

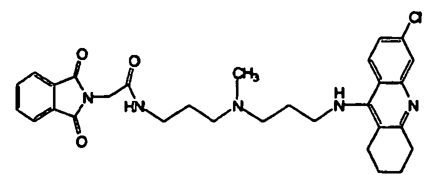

Reagents: *N*-phthaloglycine (160 mg, 0.78 mmol), THF anhydrous (10 ml), 1,1'-carbonyldiimidazol (126.5 mg, 0.78 mmol), and 6-chloro-9-(6-aminohexylamino)- 1,2,3,4-tetrahydroacridine (260 mg, 0.78 mmol).

Purification: silica gel column chromatography using DCM/ MeOH/ NH₃ (20:1:0.5%). Yellow syrup, yield: 150 mg (37%).
¹H-NMR (CDCl₃, 400MHz, δ ppm): 7.81 (d, 1H, *J*=8.9 Hz), 7.73 (d, 1H, *J*=2.3 Hz), 7.68 (dd, 2H, *J*=5.4 Hz, *J*=3.1 Hz), 7.58 (dd, 2H, *J*=5.4 Hz, *J*=3.1 Hz), 7.13 (dd, 1H, *J*=8.9 Hz, *J*=2.0 Hz), 4.21 (s, 2H), 3.52-3.47 (m, 2H), 3.33 (brs, 2H), 3.28 (c, 2H, *J*=6.6 Hz), 3.24-3.21 (brs, 2H), 2.90 (brs, 2H), 2.34 (brs, 2H), 2.09 (s, 3H), 1.72 (m, 4H), 1.69 (q, 2H, *J*=6.6 Hz), 1.60 (q , 2H, *J*=6.6 Hz).
¹³C-NMR (CDCl₃, 100MHz, δ ppm): 167.5, 166.0, 157.6, 151.5, 146.0, 134.8, 134.7, 134.0, 131.7, 125.4, 124.8, 124.3, 123.3, 117.3, 114.6, 48.9, 40.8, 39.4, 32.8, 31.4, 29.3, 26.2, 25.6, 24.5, 22.7, 22.2.
ESI-MS[M+H⁺]⁺519.

### Example 12.- N-[6-(6-Chloro-1,2,3,4-tetrahydro-acridin-9-ylamino)-hexyl]-2-(1,3-dioxo-1,3-dihydro-isoindol-2-yl)-acetamide

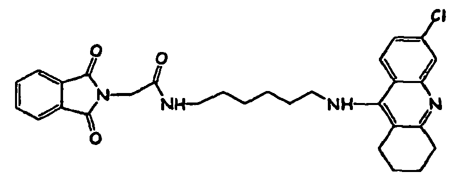

Reagents: *N*-phthaloglycine (76 mg, 0.34 mmol), THF anhydrous (8 ml), 1,1'-carbonyldiimidazol (55.1 mg, 0.34 mmol), and 6-chloro-9-(6-aminohexylamino)- 1,2,3,4-tetrahydroacridine (200 mg, 0.34 mmol).

Purification: silica gel column chromatography using DCM/ MeOH/ NH₃ (20:1:0.5%). Yellow syrup, yield: 60 mg (32%).
¹H-NMR (CDCl₃, 400MHz, δ ppm): 7:92 (d, 1H, *J*=8.9 Hz), 7.89 (d, 1H, *J*=2.3 Hz), 7.81 (dd, 2H, *J*=5.4 Hz, *J*=3.1 Hz), 7.70 (dd, 2H, *J*=5.4 Hz, *J*=3.1 Hz), 7.23 (dd, 1H, *J*=8.9 Hz, *J*=2.0 Hz), 6.04 (brs, 1H ), 4.33 (s, 2H), 3.53-3.51 (m, 2H), 3.28 (c, 2H, *J*=6.6 Hz), 3.04 (brs, 2H), 2.60 (brs, 2H), 1.90 (m, 4H), 1.70-1.66 (m , 2H), 1.55-1.52 (m, 2H), 1.40-1.36(m ,2H).
¹³C-NMR (CDCl₃, 100MHz, δ ppm): 167.5, 166.0, 157.3, 151.8, 145.4, 134.8, 133.9, 131.7, 124.9, 124.1, 123.1, 122.9, 117.0, 114.4, 56.2, 56.1, 48.8, 42.3, 40.8, 38.8, 32.3, 27.7, 26.1, 24.6, 22.7, 22.1.
ESI-MS[M+H⁺]⁺548.

### BIOLOGICAL EVALUATION

### ACHE INHIBITION (FROM HUMAN ERYTHROCYTES)

The method of Ellman *et al.* (Ellman, G.L.; Courtney, K.D.; Andres, B.; Featherstone, R.M. *Biochem. Pharmacol.* 1961, *7,* 88-95) was followed. The assay solution consisted of 0.1 M phosphate buffer pH 8, 200 µM 5,5'-dithiobis(2-nitrobenzoic acid) (DTNB, Ellan's reagent), 0.02 unit/ml AChE (Sigma Chemical Co. from human erythrocytes), and 400 µM acetylthiocholine iodide as the substrate of the enzymatic reaction. The compounds tested were added to the assay solution and pre incubated with the enzyme for 10 min at 30°C. After that period, the substrate was added. The absorbance changes at 412 nm were recorded for 5 min with a Perkin-Elmer 550 SE UV/VIS spectrometer, the reaction rates were compared, and the percent inhibition due to the presence of test compounds was calculated. The IC₅₀ is defined as the concentration of each compound that reduces a 50% the enzymatic activity with respect to that without inhibitors.

**Table 1: Human Erythrocytes AChE inhibition**

| Compound number | Structure | IC₅₀ (nM) |
|---|---|---|
| 1 | 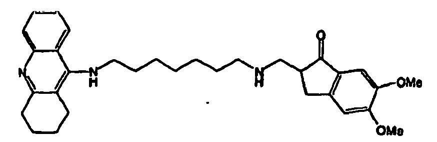 | 25 |
| 2 | 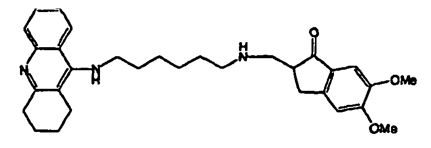 | 100 |

### ACHE INHIBITION (FROM BOVINE ERYTHROCYTES)

AChE inhibitory activity was evaluated at 25°C by the colorimetric method reported by Ellman (Ellman, G.L.; Courtney, K.D.; Andres, B.; Featherstone, R.M. *Biochem. Pharmacol.* 1961, *7*, 88-95). The assay solution consisted on 0.02 unit/ml AChE from bovine erythrocytes, 0.1 M sodium phosphate buffer pH 8, 0.3 mM 5,5'-dithiobis (2-nitrobenzoic acid) (DTNB, Ellman-s reagent), and 0.5 mM acetylthiocholine iodide as the substrate of the enzymatic reaction. Enzyme activity was determined by measuring the absorbance at 405 nm during 10 minutes with a Fluostar optima plate reader (BMG). The tested compounds were preincubated with the enzyme for 10 minutes at 30°C. In this conditions, compound 16 showed an IC₅₀ value of 2.03 E-07 M.

### NEURONAL ACHE ACTIVITY

Acetylcholinesterase (AChE) enzyme preparations were obtained from SH-SY5Y, SK-N-SH and N2A cells.

CELL CULTURE: The human neuroblastoma cell line SH-SY5Y was cultured in minimum essential medium, Han's F12 medium, supplemented with 10% fetal bovine serum and 1% penicillin/streptomycin, and grown in a 5% CO₂ humidified incubator at 37°C. The human neuroblastoma cell line SK-N-SH was cultured in RPMI 1640 medium, supplemented with 10% fetal bovine serum and 1% penicillin/streptomycin, and grown in a 5% CO₂ humidified incubator at 37°C. The rat neuroblastoma cell line N2A was cultured in DULBECCO'S MOD EAGLE medium, supplemented with 10% fetal bovine serum and 1% penicillin/streptomycin, and grown in a 5% CO₂ humidified incubator at 37°C. The cells were plated at 250·10³ cells for each reaction, at least, 48 hours before the AChE activity measure. Cells were washed and harvested in 0.1 M sodium phosphate buffer pH 8, at 4°C.

INHIBITION OF ACHE: AChE inhibitory activity was evaluated at 25°C by the colorimetric method reported by Ellman (Ellman, G.L.; Courtney, K.D.; Andres, B.; Featherstone, R.M. *Biochem. Pharmacol.* 1961, *7,* 88-95). The assay solution consisted of AChE from neuronal cells, 0.1 M phosphate buffer pH 8, 0.3 mM 5,5'-dithiobis (2-nitrobenzoic acid) (DTNB, Ellman-s reagent), and 0.5 mM acetylthiocholine iodide as the substrate of the enzymatic reaction. Enzyme activity was determined by measuring the absorbance at 405 nm during 10 minutes with a Fluostar optima plate reader (BMG).The tested compounds were preincubated with the enzyme for 10 minutes at 30°C. The reaction rate was calculated with, at least, triplicate measurements, and the percent inhibition due to the presence of test compound was calculated relative to the compound-free control. The compound concentration producing 50% of AChE inhibition (IC₅₀) was determined.

| | | N2A (rat neuroblastoma) | IC50 (M) SK-N-SY (human neuroblastoma) | SH-SY5Y (human neuroblastoma) |
|---|---|---|---|---|
| | 1 | 2,09E-07 | 1,09E-07 | 4,35E-06 |
| AChE inhibitors | | | | |
| | Tacrine | 3,95E-07 | 3,03E-07 | 3,91E-07 |

### INHIBITION OF β-AMYLOID AGGREGATION:

The generation of AChE-Aβ complexes were carried out as described previously [39,40]. Stock solutions of Aβ₁₋₄₀ at 3.5 mM were prepared in DMSO. The amount of peptide used in the assays was 0.1 mM. Human recombinant AChE (Sigma-Aldrich) was used at a molar ratio Aβ-AChE 200:1. For the aggregation studies the peptide was mixed with the appropriate amount of AChE in PBS pH 7.4 and stirred for 48 hours in a microtiter plate at room temperature. The fibrils obtained were characterized by Congo Red (CR) binding.

For the inhibition of β-amyloid aggregation, the compounds tested were used at the IC₅₀ defined in the previous paragraph of the biological evaluation. Propidium iodide 50µM for comparison.

To quantify the amount of fibrils aggregated, the binding to CR was done as described by Klunk (Klunk, WE.; Pettegrew, JW.; Abraham, DJ. *J. Hystochem. Cytochem.,* 1989, 8, 1293-1297). Briefly, 5.5 µl aliquot of the aggregation mixture were added to 132 µl of a solution of 25 M CR (100mM phosphate buffer pH 7.4, 150mM NaCl) and incubated for 30 minutes at room temperature. Absorbance was measured at 480 and 540nm. The CR binding was estimated by CR (M)= (A₅₄₀/25295)-(A₄₈₀/46306).

In the conditions above described, the indanone-tacrine derivative 1 showed a 18.7% reduction of the amyloid-AChE complex aggregation. The peripheral inhibitor propidium reduces the aggregation of the β-amyloid-AChE complex by 18.1 %. This compound was used as standard of reference.

### ACETYLCHOLINESTERASE (ACHE) INHIBITION (FROM BOVINE ERYTHROCYTES)

AChE inhibitory activity was evaluated at 30°C by the colorimetric method reported by Ellman [Ellman, G.L.; Courtney, K.D.; Andres, B.; Featherstone, R.M. *Biochem. Pharmacol.* 1961, 7, 88-95]. The assay solution consisted of 0.1 M phosphate buffer pH 8, 0.3 mM 5,5'-dithiobis (2-nitrobenzoic acid) (DTNB, Ellman's reagent), 0.02 units AChE (Sigma Chemical Co. from bovine erythrocytes), and 0.5 mM acetylthiocholine iodide as the substrate of the enzymatic reaction. The compounds tested were added to the assay solution and preincubated with the enzyme for 5 min at 30°C. After that period, the substrate was added. The absorbance changes at 405 nm were recorded for 5 min with a microplate reader Digiscan 340T, the reaction rates were compared, and the percent inhibition due to the presence of test compounds was calculated. The reaction rate was calculated with, at least, triplicate measurements, and the percent inhibition due to the presence of test compound was calculated relative to the compound-free control. The compound concentration producing 50% of AChE inhibition (IC₅₀) was determined. The results are shown in table 2.

### BUTYRYLCHOLINESTERSAE (BUCHE) INHIBITION (FROM HUMAN SERUM)

BuChE inhibitory activity was evaluated at 30°C by the colorimetric method reported by Ellman [Ellman, G.L.; Courtney, K.D.; Andres, B.; Featherstone, R.M. *Biochem. Pharmacol.* 1961, *7,* 88-95]. The assay solution consisted of 0.01 units BuChE from human serum, 0.1 M sodium phosphate buffer pH 8, 0.3 mM 5,5'-dithiobis (2-nitrobenzoic acid) (DTNB, Ellman's reagent), and 0.5 mM butyrylthiocholine iodide as the substrate of the enzymatic reaction. Enzyme activity was determined by measuring the absorbance at 405 nm during 5 minutes with a microplate reader Digiscan 340T. The tested compounds were preincubated with the enzyme for 10 minutes at 30°C. The reaction rate was calculated with, at least, triplicate measurements. The IC₅₀ is defined as the concentration of each compound that reduces a 50% the enzymatic activity with respect to that without inhibitors. The results are shown in table 2.

### TOXICITY MEASUREMENT

The cytotoxicity effect of the molecules was tested in the human neuroblastoma cell line SH-SY5Y. These cells were cultured in 96-well plates in minimum essential medium, Han's F12 medium, supplemented with 10% fetal bovine serum, 1% glutamine and 1% penicillin/streptomycin, and grown in a 5% CO₂ humidified incubator at 37°C.

The cells were plated at 10⁴ cells for each well, at least, 48 hours before the toxicity measure. Cells were exposed for 24 hours to the compounds at different concentrations (from 10⁻⁵ to 10⁻⁹), quantitative assessment of cell death was made by measurement of the intracellular enzyme lactate dehydrogenase (LDH) (citotoxicity detection kit, Roche). The cuantity of LDH was evaluated in a microplate reader Anthos 2010, at 492 and 620 nm. Controls were taken as 100% viability. The results are shown in table 2.

### PROPIDIUM COMPETITION

Propidium exhibits an increase in fluorescence on binding to AchE peripheral site, making it a useful probe for competitive ligand binding to the enzyme.

Fluorescence was measured in a Fluostar optima plate reader (BMG). Measurements were carried out in 100 µl solution volume, in 96-well plates. The buffer used was 1 mM Tris/HCl, pH 8.0. 10 M AchE was incubated, at least 6 hours, with the molecules at different concentrations. 20 µM propidium iodide was added 10 min before fluorescence measurement. The excitation wavelength was 485 nm, and that of emission, 620 nm. The results are shown in table 2.

### EFFECTS ON CALCIUM CHANNELS

We have studied if these compounds are able to block calcium channels. Calcium entry in SH-SY5Y cells was stimulated with 60 mM K⁺ in 96-well plates. Cells were plated at 5·10⁴ for each well, at least, 48 hours before the experiment. For measurement of intracellular calcium, SH-SY5Y were washed with Krebs/HEPES buffer, pH 7.4. and cells were loaded with the calcium indicator dye Fluo-4 (Molecular Probes) for 40 min at 37°C followed by 15 min post incubation at room temperature. Intracellular calcium was measured fluorometrically in a Fluostar optima plate reader (BMG), with excitation wavelengths set at 485 nm and emission at 520 nm. The tested compounds were preincubated with the cells for 10 minutes before the depolarization with K⁺. The results are shown in table 2.

### ANTIOXIDANT ACTIVITY

In order to evaluate if these compounds have antioxidant properties cells SH-SY5Y were exposed 24 hours to 100 M H₂O₂, previously these cells were pretreated for 1 hour with the molecules at different concentrations (from 10⁻⁵ to 10⁻⁹). The antioxidant activity is evaluated measuring cellular viability, using the LDH assay. Cells SH-SY5Y were cultured in 96-well plates as toxicity experiments. The results are shown in table 2.

### EFFECTS ON β-AMILOID TOXICITY

We have tested if some of these molecules interfere with amyloid peptide toxicity. Cells SH-SY5Y were cultured in 96-well plates as toxicity experiments. Cells were pretreated for 1 hour with the compounds at different concentrations (from 10⁻⁵ to 10⁻⁹), immediately neuronal death is induced adding synthetic peptide β-amyloid, fragment 25-35 (A ₂₅₋₃₅) at 200 µM. 24 hours later cell viability was assessed with the LDH assay; results are reported relative to untreated wells. The results are shown in table 2.

**TABLE 2**

| | | | | |
|---|---|---|---|---|
| Comp no. | structure | IC₅₀ AchE (nM) | IC₅₀ BuChE (nM) | Toxicity (µM) |
| 3 | 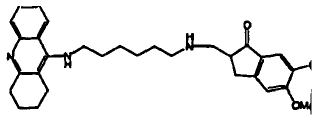 | 500 | 100 | 10 |
| 4 | 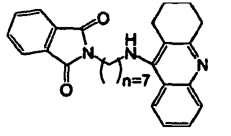 | 3000 | 10 | >100 |
| 5 | 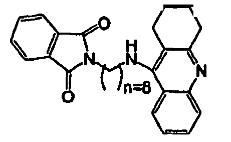 | 90 | 30 | 5 |
| 6 | 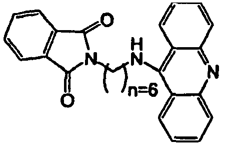 | 900 | 8 | >100 |
| 7 | 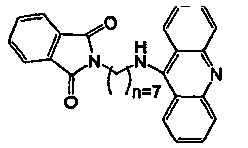 | 1500 | 700 | >100 |
| 8 | 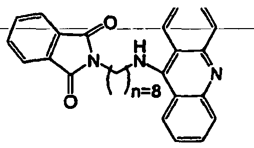 | 1000 | 1000 | 100 |
| 9 | 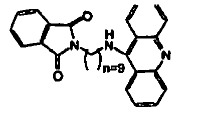 | 2 | 90 | >100 |
| 10 | 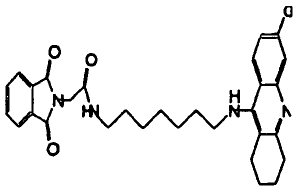 | 20 | 650 | >100 |
| 11 | 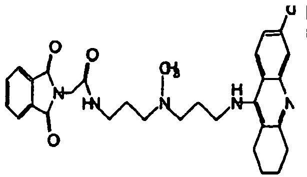 | 3 | 75 | >100 |
| 12 | 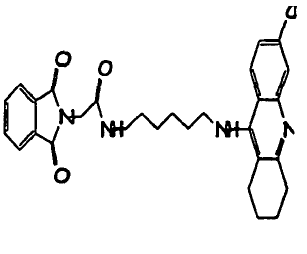 | 10 | 200 | >100 |

| Comp . no | Toxicity (µM) | Propidium competition (µM) | Calcium channel blockade (µM) | Antioxidant activity (µM) | inhibition of Aβ₍₂₅₋₃₅₎ toxicity (µM) |
|---|---|---|---|---|---|
| 3 | 10 | >100 | NO | NO | |
| 4 | >100 | 10 | NO | NO | |
| 5 | 5 | >100 | NO | NO | |
| 6 | > 100 | 10 | NO | NO | |
| 7 | >100 | 100 | 10 | NO | 1 |
| 8 | 100 | >100 | NO | NO | |
| 9 | >100 | 1 | NO | NO | |
| 10 | >100 | 10 | NO | NO | |
| 11 | >100 | 10 | NO | NO | |
| 12 | > 100 | 100 | NO | NO | |

## Claims

1. A compound represented by the general formula (I) 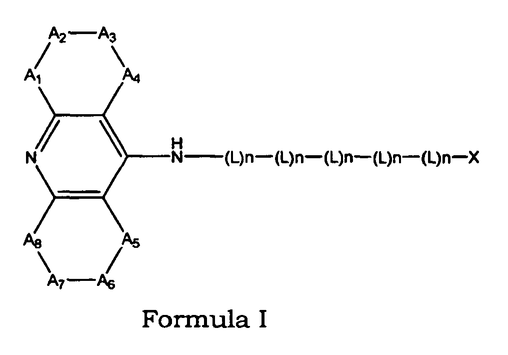 wherein:
X is the following radical: 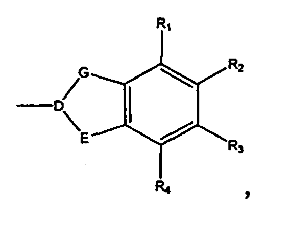
L is independently selected from -C(R)(R")-, -CO-, -O- or -NR'-
n is zero, one, two, three, four, five, six, seven, eight, nine or ten
R and R" are independently selected from hydrogen, alkyl, aryl, heteroaryl, halo, haloalkyl, alkoxy, hydroxyl, nitro and alkylthio
D is independently selected from -C(R₉)-, =C-, or -N-
A₁, A₂, A₃, A₄, A₅, A₇, A₈, E and G are independently selected from -CO-, -C(R₁₀)(R₁₁)-, =C(Rio)-, -N(R₁₂)-, =N-, -O-, -S(O)ₜ-
R₁, R₂, R₃, R₄, R₉, Rio and R₁₁ are independently selected from hydrogen, alkyl, alkoxy, hydroxyl, alkylthio, cycloalkyl, haloalkyl, halo, aryl, -(Z)ₙ-, aryl, heteroaryl, -O(R₇), -C(O)R₇, -C(O)OR₇, -S(O)ₜ, cyano, nitro and mercapto, aryl substituted by alkyl, alkoxy, hydroxy, halo, haloalkyl, nitro or alkylthio; and heteroaryl substituted by alkyl, alkoxy, hydroxy, halo, haloalkyl, nitro or alkylthio
R₁₂ is independently selected from hydrogen, alkyl, alkoxy, hydroxyl, cycloalkyl, haloalkyl, aryl, heteroaryl, aryl substitued by alkyl, alkoxy, hydroxy, halo, haloalkyl or alkylthio; and heteroaryl substituted by alkyl, alkoxy, hydroxy, halo, haloalkyl, nitro or alkylthio.Z is independently selected from -C(R₇)(R₈)-, -C(O)-, -O-, -C(=NR₇)-, -S(O)ₜ, N(R₇)-
R₇ and R₈ are independently selected from hydrogen, alkyl, alkoxy, alkylthio, cycloalkyl, haloalkyl, halo, aryl, heteroaryl, cyano, nitro, mercapto, aryl substituted by alkyl, alkoxy, hydroxy, halo, haloalkyl, nitro or alkylthio; and heteroaryl substituted by alkyl, alkoxy, hydroxy, halo, haloalkyl, nitro or alkylthio
t is zero, one or two;
with the exclusion of the following two compounds:

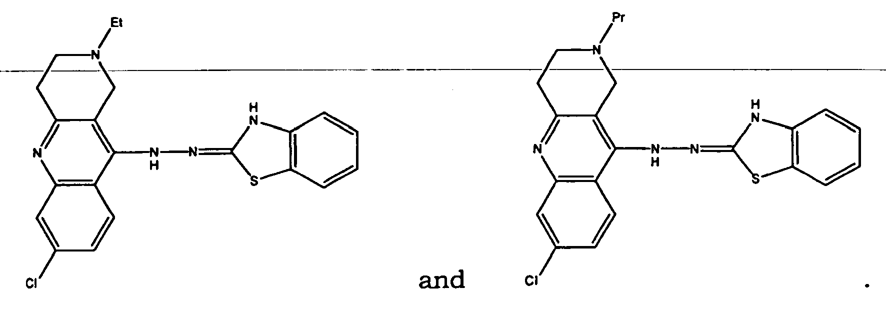

2. A compound according to claim 1, wherein D is -CH-, =C- or -N;

3. A compound according to any one of claims 1 and 2, wherein E is -CO-, -CH₂-. =CH-, =N- -O- or -S-.

4. A compound according to any one of claims 1 to 3, wherein G is -CO-, -CH₂-. =CH-, or =N-.

5. A compound according to any one of claims 1 to 4, wherein R₁ to R₄ are hydrogen.

6. A compound according to claim 1, wherein X is selected from phthalimidyl (1,3-dioxo-1,3-dihydro-isoindol-2-yl), indol-2-yl, 1-indanon-2-yl, benzimidazol-2-yl, indadion-2-yl, indazol-2-yl, benzofuran-2-yl, benzothiophen-2-yl, or benzotriazol-2-yl.

7. A compound according to claim 1 wherein X is phthalimidyl (1,3-dioxo-1,3-dihydro-isoindol-2-yl) and the cyclic part of formula I represents 9-acridinyl, 1,2,3,4-tetrahydro-acridin-9-yl or 6-chloro-1,2,3,4-tetrahydro-acridin-9-yl.

8. A compound according to claim 1 which is:
- 2-[6-(acridin-9-ylamino)-hexyl]-isoindole-1,3-dione (6),
- 2-[7-(acridin-9-ylamino)-heptyl]-isoindole-1,3-dione (7),
- 2-[8-(acridin-9-ylamino)-octyl]-isoindole-1,3-dione (8),
- 2-[9-(acridin-9-ylamino)-nonyl]-isoindole-1,3-dione (9),
- N-[7-(6-Chloro-1,2,3,4-tetrahydro-acridin-9-ylamino)-heptyl]-2-(1,3-dioxo-1,3-dihydro-isoindol-2-yl)-acetamide (10),
- N-(3-{[3-(6-Chloro-1,2,3,4-tetrahydro-acridin-9-ylamino)-propyl]-methyl-amino}-propyl)-2-(1,3-dioxo-1,3-dihydro-isoindol-2-yl)-acetamide (11),
- N-[6-(6-Chloro-1,2,3,4-tetrahydro-acridin-9-ylamino)-hexyl]-2-(1,3-dioxo-1,3-dihydro-isoindol-2-yl)-acetamide (12),
- 2-[6-(1,2,3,4-Tetrahydro-acridin-9-ylamino)-hexylamino]-indan-1,3-dione (3),
- 2-[7-(1,2,3,4-Tetrahydro-acridin-9-ylamino)-heptyl]-isoindole-1,3-dione (4), or
- 2-[8-(1,2,3,4-Tetrahydro-acridin-9-ylamino)-octyl]-isoindole-1,3-dione (5).

9. A compound according to claim 1 wherein X is 1-indanon-2-yl and the cyclic part of formula I represents 1,2,3,4-tetrahydro-acridin-9-yl.

10. A compound according to claim 9 which is:
- 5,6-Dimethoxy-2-{[7-(1,2,3,4-tetrahydro-acridin-9-ylamino)-heptylamino]-methyl}-indan-1-one (1) or
- 5,6-Dimethoxy-2-{[6-(1,2,3,4-tetrahydro-acridin-9-ylamino)-hexylamino]-methyl}-indan-1-one (2).

11. A pharmaceutical formulation containing as active ingredient a compound as defined in any of claims 1 to 10.

12. Compound according to any of claims 1 to 10 for use as a medicament

13. Compound according to any of claims 1 to 10 for use in treating cognitive disorders as senile dementia, cerebrovascular dementia, mild cognition impairment, attention deficit disorder, and/or neurodegenerative dementing disease with aberrant protein aggregations as specially Alzheimer's disease, Parkinson disease, ALS, or prion diseases, as Creutzfeldt-Jakob disease or Gerstmann-Straussler-Scheinher disease.

14. Use of a compound according to any of claims 1 to 10 for preparing a medicament.

15. Use of a compound according to any of claims 1 to 10 for the manufacture of a medicament for treating cognitive disorders as senile dementia, cerebrovascular dementia, mild cognition impairment, attention deficit disorder, and/or neurodegenerative dementing disease with aberrant protein aggregations as specially Alzheimer's disease, Parkinson disease, ALS, or prion diseases, as Creutzfeldt-Jakob disease or Gerstmann-Straussler-Scheinher disease.

## Patentansprüche

1. Verbindung, dargestellt durch die allgemeine Formel (I) 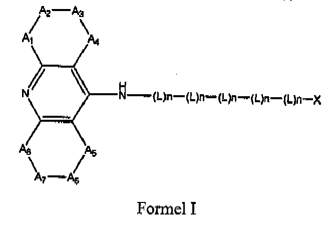 wobei:
X der folgende Rest ist: 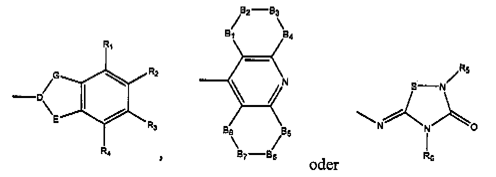
L unabhängig aus -C(R)(R")-, -CO-, -O- oder -NR'- ausgewählt ist
n null, eins, zwei, drei, vier, fünf, sechs, sieben, acht, neun oder zehn ist
R und R" unabhängig aus Wasserstoff, Alkyl, Aryl, Heteroaryl, Halogen, Halogenalkyl, Alkoxy, Hydroxyl, Nitro und Alkylthio ausgewählt sind
D unabhängig aus -C(R₉)-, =C- oder -N- ausgewählt ist
A₁, A₂, A₃, A₄, A₅, A₇, A₈, E und G unabhängig aus -CO-, -C(R₁₀)(R₁₁)-, =C(R₁₀)-, -N(R₁₂)-, =N-, -O-, -S(O)ₜ- ausgewählt sind
R₁, R₂, R₃, R₄, R₉, R₁₀ und R₁₁ unabhängig aus Wasserstoff, Alkyl, Alkoxy, Hydroxyl, Alkylthio, Cycloalkyl, Halogenalkyl, Halogen, Aryl, -(Z)ₙ-, Aryl, Heteroaryl, -O(R₇), -C(O)R₇, -C(O)OR₇, -S(O)ₜ, Cyano, Nitro und Mercapto, Aryl, substituiert durch Alkyl, Alkoxy, Hydroxy, Halogen, Halogenalkyl, Nitro oder Alkylthio; und Heteroaryl, substituiert durch Alkyl, Alkoxy, Hydroxy, Halogen, Halogenalkyl, Nitro oder Alkylthio, ausgewählt sind
R₁₂ unabhängig aus Wasserstoff, Alkyl, Alkoxy, Hydroxyl, Cycloalkyl, Halogenalkyl, Aryl, Heteroaryl, Aryl, substituiert durch Alkyl, Alkoxy, Hydroxy, Halogen, Halogenalkyl oder Alkylthio; und Heteroaryl, substituiert durch Alkyl, Alkoxy, Hydroxy, Halogen, Halogenalkyl, Nitro oder Alkylthio, ausgewählt ist.
ist Z unabhängig aus -C(R₇)(R₈)-, -C(O)-, -O-, -C(=NR₇)-, -S(O)₂, N(R₇)- ausgewählt
R₇ und R₈ unabhängig aus Wasserstoff, Alkyl, Alkoxy, Alkylthio, Cycloalkyl, Halogenalkyl, Halogen, Aryl, Heteroaryl, Cyano, Nitro, Mercapto, Aryl, substituiert durch Alkyl, Alkoxy, Hydroxy, Halogen, Halogenalkyl, Nitro oder Alkylthio; und Heteroaryl, substituiert durch Alkyl, Alkoxy, Hydroxy, Halogen, Halogenalkyl, Nitro oder Alkylthio, ausgewählt sind
t null, eins oder zwei ist;
mit dem Ausschluss der folgenden zwei Verbindungen:

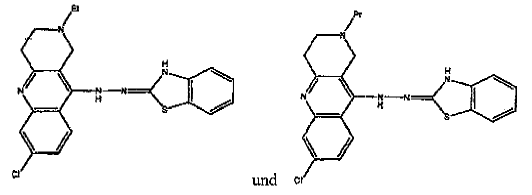

2. Verbindung nach Anspruch 1, wobei D -CH-, =C- oder -N ist;

3. Verbindung nach einem der Ansprüche 1 und 2, wobei E -CO-, -CH₂-, =CH-, =N--O- oder -S- ist.

4. Verbindung nach einem der Ansprüche 1 bis 3, wobei G -CO-, -CH₂-, =CH- oder =N-ist.

5. Verbindung nach einem der Ansprüche 1 bis 4, wobei R₁ bis R₄ Wasserstoff sind.

6. Verbindung nach Anspruch 1, wobei X aus Phthalimidyl-(1,3-Dioxo-1,3-dihydro-isoindol-2-yl), Indol-2-yl, 1-Indanon-2-yl, Benzimidazol-2-yl, Indadion-2-yl, Indazol-2-yl, Benzofuran-2-yl, Benzothiophen-2-yl oder Benzotriazol-2-yl ausgewählt ist.

7. Verbindung nach Anspruch 1, wobei X Phthalimidyl (1,3-Dioxo-1,3-dihydro-isoindol-2-yl) ist und der cyclische Teil von Formel I 9-Acridinyl, 1,2,3,4-Tetrahydro-acridin-9-yl oder 6-Chlor-1,2,3,4-tetrahydro-acridin-9-yl darstellt.

8. Verbindung nach Anspruch 1, welche:
- 2-[6-(Acridin-9-ylamino)-hexyl]-isoindol-1,3-dion (6),
- 2-[7-(Acridin-9-ylamino)-heptyl]-isoindol-1,3-dion (7),
- 2-[8-(Acridin-9-ylamino)-octyl]-isoindol-1,3-dion (8),
- 2-[9-(Acridin-9-ylamino)-nonyl]-isoindol-1,3-dion (9),
- N-[7-(6-Chlor-1,2,3,4-tetrahydro-acridin-9-ylamino)-heptyl]-2-(1,3-dioxo-1,3-dihydro-isoindol-2-yl)-acetamid (10),
- N-(3-{[3-(6-Chlor-1,2,3,4-tetrahydro-acridin-9-ylamino)-propyl]-methylamino}-propyl)-2-(1,3-dioxo-1,3-dihydro-isoindol-2-yl)-acetamid (11),
- N-[6-(6-Chlor-1,2,3,4-tetrahydro-acridin-9-ylamino)-hexyl]-2-(1,3-dioxo-1,3-dihydro-isoindol-2-yl)-acetamid (12),
- 2-[6-(1,2,3,4-Tetrahydro-acridin-9-ylamino)-hexylamino]-indan-1,3-dion (3),
- 2-[7-(1,2,3,4-Tetrahydro-acridin-9-ylamino)-heptyl]-isoindol-1,3-dion (4) oder
- 2-[8-(1,2,3,4-Tetrahydro-acridin-9-ylamino)-octyl]-isoindol-1,3-dion (5) ist.

9. Verbindung nach Anspruch 1, wobei X 1-Indanon-2-yl ist und der cyclische Teil von Formel I 1,2,3,4-Tetrahydro-acridin-9-yl darstellt.

10. Verbindung nach Anspruch 9, welche:
- 5,6-Dimethoxy-2-{[7-(1,2,3,4-tetrahydro-acridin-9-ylamino)-heptylamino]-methyl}-indan-1-on (1) oder
- 5,6-Dimethoxy-2-{[6-(1,2,3,4-tetrahydro-acridin-9-ylamino)-hexylamino]-methyl}-indan-1-on (2) ist.

11. Pharmazeutische Formulierung, enthaltend als Wirkstoff eine Verbindung wie in einem der Ansprüche 1 bis 10 definiert.

12. Verbindung nach einem der Ansprüche 1 bis 10 zur Verwendung als Medikament.

13. Verbindung nach einem der Ansprüche 1 bis 10 zur Verwendung beim Behandeln kognitiver Störungen wie senile Demenz, zerebrovaskuläre Demenz, milde Wahrnehmungsbeeinträchtigung, Aufmerkamkeits-Defizit-Erkrankung und/oder neurodegenerative dement machende Krankheit mit aberranten Proteinaggregationen wie speziell Alzheimer-Krankheit, Parkinson-Krankheit, ALS oder Prionen-Krankheiten, wie Creutzfeldt-Jakob-Krankheit oder Gerstmann-Sträussler-Scheinker-Krankheit.

14. Verwendung einer Verbindung nach einem der Ansprüche 1 bis 10 zum Herstellen eines Medikaments.

15. Verwendung einer Verbindung nach einem der Ansprüche 1 bis 10 für die Herstellung eines Medikaments zum Behandeln kognitiver Störungen wie senile Demenz, zerebrovaskuläre Demenz, milde Wahrnehrnungsbeeinträchtigung, Aufmerkamksits-Defizit-Erkrankung und/oder neurodegenerative dement machende Krankheit mit aberranten Proteinaggregationen wie speziell Alzheimer-Krankheit, Parkinson-Krankheit, ALS oder Prionen-Krankheiten, wie Creutzfeldt-Jakob-Krankheit oder Gerstmann-Sträussler-Scheinker-Krankheit.

## Revendications

1. Composé répondant à la formule générale (I) 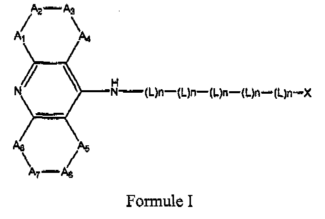 où:
X représente le radical suivant: 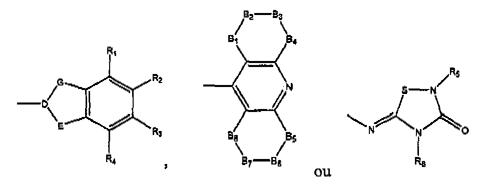
L est indépendamment sélectionné parmi -C(R)(R")-, -CO-, -O- ou -NR'-;
n est égal à zéro, un, deux, trois, quatre, cinq, six, sept, huit, neuf ou dix,
R et R" sont indépendamment sélectionnés parmi un hydrogène, alkyle, aryle, hétéroaryle, halo, haloalkyle, alkoxy, hydroxyle, nitro et alkylthio;
D est indépendamment sélectionné parmi -C(R₉)-, =C- ou -N-;
A₁, A₂ A₃, A₄, A₅, A₇, A₈, E et G sont indépendamment sélectionnés parmi -CO-, -C(R₁₀)(R₁₁)-, =C(R₁₀)-, -N(R₁₂)-, =N-, -O- et -S(O)ₜ-;
R₁, R₂, R₃, R₄, R₉, R₁₀ et R₁₁ sont indépendamment sélectionnés parmi un hydrogène, alkyle, alkoxy, hydroxyle, alkylthio, cycloalkyle, haloalkyle, halo, aryle, -(Z)ₙ-, aryle, hétéroaryle, -O(R₇), -C(O)R₇, -C(O)OR₇, -S(O)ₜ, cyano, nitro et mercapto, aryle substitué par un alkyle, alkoxy, hydroxy, halo, haloalkyle, nitro ou alkylthio et hétéroaryle substitué par un alkyle, alkoxy, hydroxy, halo, haloalkyle, nitro ou alkylthio;
R₁₂ est indépendamment sélectionné parmi un hydrogène, alkyle, alkoxy, hydroxyle, cycloalkyle, haloalkyle, aryle, hétéroaryle, aryle substitué par un alkyle, alkoxy, hydroxy, halo, haloalkyle ou alkylthio et hétéroaryle substitué par un alkyle, alkoxy, hydroxy, halo, haloalkyle, nitro ou alkylthio;
Z est indépendamment sélectionné parmi -C(R₇)(R₈)-, -C(O)-, -O-, -C(=NR₇)-, -S(O)ₜ et N(R₇)-;
R₇ et R₈ sont indépendamment sélectionnés parmi un hydrogène, alkyle, alkoxy, alkylthio, cycloalkyle, haloalkyle, halo, aryle, hétéroaryle, cyano, nitro, mercapto, aryle substitué par un alkyle, alkoxy, hydroxy, halo, haloalkyle, nitro ou alkylthio et hétéroaryle substitué par un alkyle, alkoxy, hydroxy, halo, haloalkyle, nitro ou alkylthio;
t est égal à zéro, un ou deux;
à l'exclusion des deux composés suivants:

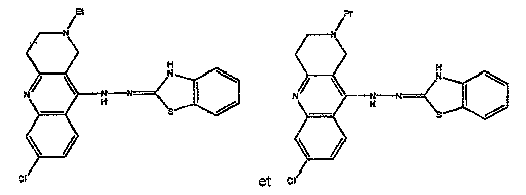

2. Composé selon la revendication 1, où D représente -CH-, =C- ou -N.

3. Composé selon l'une quelconque des revendications 1 et 2, où E représente -CO-, -CH₂-, =CH-, =N-, -O- ou -S-.

4. Composé selon l'une quelconque des revendications 1 à 3, où G représente -CO-, -CH₂-, =CH- ou =N-.

5. Composé selon l'une quelconque des revendications 1 à 4, où R₁ à R₄ représentent un hydrogène.

6. Composé selon la revendication 1, où X est sélectionné parmi les groupements suivants: phtalimidyl-(1,3-dioxo-1,3-dihydro-isoindol-2-yle), indol-2-yle, 1-indanon-2-yle, benzimidazol-2-yle, indadion-2-yle, indazol-2-yle, benzofuran-2-yle, benzothiophén-2-yle ou benzotriazol-2-yle.

7. Composé selon la revendication 1, où X représente un groupement phtalimidyl-(1,3-dioxo-1,3-dihydro-isoindol-2-yle) et la portion cyclique de la formule I correspond à un groupement 9-acridinyle, 1,2,3,4-tétrahydro-acridin-9-yle ou 6-chloro-1,2,3,4-tétrahydro-acridin-9-yle.

8. Composé selon la revendication 1, qui correspond à l'un des suivants:
- 2-[6-(Acridin-9-ylamino)-hexyl]-isoindole-1,3-dione (6),
- 2-[7-(Acridin-9-ylamino)-heptyl]-isoindole-1,3-dione (7),
- 2-[8-(Acridin-9-ylamino)-octyl]-isoindole-1,3-dione (8),
- 2-[9-(Acridin-9-ylamino)-nonyl]-isoindole-1,3-dione (9),
- N-[7-(6-Chloro-1,2,3,4-tétrahydro-acridin-9-ylamino)-heptyl]-2-(1,3-dioxo-1,3-dihydro-isoindol-2-yl)-acétamide (10),
- N-(3-{[3-(6-Chloro-1,2,3,4-tétrahydro-acridin-9-ylamino)-propyl]-méthyl-amino}-propyl)-2-(1,3-dioxo-1,3-dihydro-isoindol-2-yl)-acétamide (11),
- N-[6-(6-Chloro-1,2,3,4-tétrahydro-acridin-9-ylamino)-hexyl]-2-(1,3-dioxo-1,3-dihydro-isoindol-2-yl)-acétamide (12),
- 2-[6-(1,2,3,4-Tétrahydro-acridin-9-ylamino)-hexylamino]-indan-1,3-dione (3),
- 2-[7-(1,2,3,4-Tétrahydro-acridin-9-ylamino)-heptyl]-isoindole-1,3-dione (4), ou
- 2-[8-(1,2,3,4-Tétrahydro-acridin-9-ylamino)-octyl]-isoindole-1,3-dione (5).

9. Composé selon la revendication 1, où X représente un groupement 1-indanon-2-yle et la portion cyclique de la formule I correspond à un groupement 1,2,3,4-tétrahydro-acridin-9-yle.

10. Composé selon la revendication 9, qui correspond au:
- 5,6-Diméthoxy-2-{[7-(1,2,3,4-tétrahydro-acridin-9-ylamino)-heptylamino]-méthyl}-indan-1-one (1) ou au
- 5,6-Diméthoxy-2-{[6-(1,2,3,4-tétrahydro-acridin-9-ylamino)-hexylamino]-méthyl}-indan-1-one (2).

11. Formulation pharmaceutique qui contient comme principe actif un composé tel que défini à l'une quelconque des revendications 1 à 10.

12. Composé selon l'une quelconque des revendications 1 à 10, qui est destiné à être utilisé comme un médicament.

13. Composé selon l'une quelconque des revendications 1 à 10, qui est destiné à être utilisé dans le traitement de troubles cognitifs tels que la démence sénile, la démence vasculaire, l'insuffisance cognitive légère, le déficit de l'attention et/ou la démence neurodégénérative avec agrégations de protéines aberrantes comme, en particulier, dans la maladie d'Alzheimer, la maladie de Parkinson et la sclérose latérale amyotrophique, ou de maladies à prions comme la maladie de Creutzfeldt-Jakob ou le syndrome de Gerstmann-Sträussler-Scheinker.

14. Utilisation d'un composé selon l'une quelconque des revendications 1 à 10 dans la préparation d'un médicament.

15. Utilisation d'un composé selon l'une quelconque des revendications 1 à 10 dans la fabrication d'un médicament indiqué dans le traitement de troubles cognitifs tels que la démence sénile, la démence vasculaire, l'insuffisance cognitive légère, le déficit de l'attention et/ou la démence neurodégénérative avec agrégations de protéines aberrantes comme, en particulier, dans la maladie d'Alzheimer, la maladie de Parkinson et la sclérose latérale amyotrophique, ou de maladies à prions comme la maladie de Creutzfeldt-Jakob ou le syndrome de Gerstmann-Sträussler-Scheinker.
